# EUROPÄISCHE PATENTSCHRIFT

(11) **EP 1 646 365 B1**
(45) Veröffentlichungstag und Bekanntmachung des Hinweises auf die Patenterteilung: **14.01.2009**
(21) Anmeldenummer: 04740685.5
(22) Anmeldetag: 06.07.2004
(51) Int. Cl.: A61K 9/14, A61K 31/46

(54) **PULVERFORMULIERUNGEN FÜR DIE INHALATION ENTHALTEND EIN NEUES ANTICHOLINERGIKUM**
POWDERY FORMULATIONS FOR INHALATION, CONTAINING A NOVEL ANTICHOLINERGIC AGENT
COMPOSITIONS PULVERULENTES POUR L'INHALATION, CONTENANT UN NOUVEL AGENT ANTICHOLINERGIQUE

(30) Priorität: 11.07.2003 DE 10331350
(43) Veröffentlichungstag der Anmeldung: 19.04.2006
(73) Patentinhaber: BOEHRINGER INGELHEIM INTERNATIONAL GMBH, D-55216 Ingelheim (DE); Boehringer Ingelheim Pharma GmbH & Co. KG, 55216 Ingelheim (DE)
(72) Erfinder: SCHMIDT, Friedrich, 55218 Ingelheim (DE); TRUNK, Michael, 55218 Ingelheim (DE)
(74) Vertreter: Hammann, Heinz
(86) Internationale Anmeldenummer: PCT/EP2004/007357
(87) Internationale Veröffentlichungsnummer: WO 2005/007134

(56) Entgegenhaltungen:
- WO-A-02/30390
- WO-A-02/32899

## Beschreibung

Die Erfindung betrifft Pulverformulierungen für die Inhalation, enthaltend als alleinigen Wirkstoff ein Hydrat eines Anticholinergikums der Formel **1**. 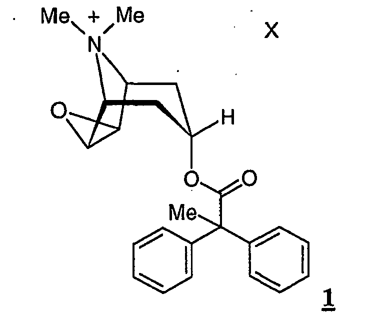 worin X⁻ für ein Anion mit den in der Beschreibung und in den Ansprüchen genannten Bedeutungen stehen kann, Verfahren zu deren Herstellung sowie deren Verwendung zur Herstellung eines Arzneimittels zur Behandlung von Atemwegserkrankungen, insbesondere zur Behandlung von COPD (chronic obstructive pulmonary disease = chronisch obstruktive Lungenerkrankung) und Asthma.

### Hintergrund der Erfindung

Die Verbindungen der Formel **1** sind aus der WO 02/32899 bekannt. Sie besitzen wertvolle pharmakologische Eigenschaften und können als hochwirksame Anticholinergika bei der Therapie von Atemwegserkrankungen, insbesondere bei der Therapie entzündlicher und/oder obstruktiver Atemwegserkrankungen, insbesondere bei der Therapie von Asthma oder COPD (chronic obstructive pulmonary disease = chronisch obstruktive Lungenerkrankung) einen therapeutischen Nutzen entfalten.

Bei der Behandlung vorstehender Erkrankungen bietet sich die inhalative Applikation des Wirkstoffs an. Neben der inhalativen Applikation von broncholytisch wirksamen Verbindungen in Form von Dosieraerosolen und Lösungen zur Inhalation kommt der Applikation von wirkstoffhaltigen Inhalationspulvern besondere Bedeutung zu.

Bei Wirkstoffen, die eine besonders hohe Wirksamkeit aufweisen, sind pro. Einzeldosis zur Erzielung des therapeutisch erwünschten Effekts nur geringe Mengen des Wirkstoffs erforderlich. In solchen Fällen ist es notwendig, zur Herstellung des Inhalationspulvers den Wirkstoff mit geeigneten Hilfsstoffen zu verdünnen. Aufgrund des hohen Anteils an Hilfsstoff werden die Eigenschaften des Inhalationspulvers maßgeblich durch die Wahl des Hilfsstoffs beeinflußt. Bei der Wahl des Hilfsstoffs kommt dessen Korngröße eine besondere Bedeutung zu. Je feiner der Hilfsstoff desto schlechter sind in der Regel dessen Fließeigenschaften. Gute Fließeigenschaften sind allerdings Voraussetzung für eine hohe Dosiergenauigkeit bei der Abfüllung und Abteilung der einzelnen Präparatedosen, wie etwa bei der Herstellung von Kapseln (Inhaletten) zur Pulverinhalation oder der Dosierung eines Einzelhubes durch den Patienten vor der Anwendung eines Mehrdosisinhalators. Des weiteren ist die Korngröße des Hilfsstoffs von großer Bedeutung für das Entleerungsverhalten von Kapseln in einem Inhalator bei der Anwendung. Es hat sich ferner gezeigt, daß die Korngröße des Hilfsstoffs starken Einfluß auf den ausgebrachten inhalierbaren Wirkstoffanteil des Inhalationspulvers hat. Unter inhalierbarem bzw. inhalierfähigem Wirkstoffanteil werden die Teilchen des Inhalationspulvers verstanden, die beim Inhalieren mit der Atemluft tief in die Verästelungen der Lunge transportiert werden. Die hierzu erforderliche Teilchengröße liegt zwischen 1 und 10µm, vorzugsweise unter 6 µm.

Es ist Aufgabe der Erfindung, den Wirkstoff der Formel **1** enthaltende Inhaltionspulver bereitzustellen, welche bei guter Dosiergenauigkeit (betreffend die pro Inhalationsvorgang ausgebrachte und lungengängige Wirkstoffmenge) und geringer chargenweisen Variabilität die Applikation des Wirkstoffs mit hohem inhalierfähigen Anteil erlaubt. Es ist ferner Aufgabe der vorliegenden Erfindung, ein den Wirkstoff der Formel **1** enthaltendes Inhaltionspulver bereitzustellen, welches, sofern die Applikation des Pulvers mittels pulverhaltiger Kapseln erfolgt, ein gutes Entleerungsverhalten der Kapseln gewährleistet.

Ferner ist es Aufgabe der Erfindung, ein Inhalationspulver bereitzustellen, welches durch ein hohes Maß an Homogenität der Pulvermischung und eine geringe Schwankung im Dispergierverhalten von Pulver- zu Pulvercharge gekennzeichnet ist. Die Homogenität der Pulvermischung wie auch gering schwankende Dispergiereigenschaften tragen entscheidend dazu bei, daß die Freisetzung des inhalierfähigen Anteils des Wirkstoffs reproduzierbar in gleichbleibend hohen Mengen und somit möglichst geringer Variabilität erfolgt. Dementsprechend zielt die vorliegende Erfindung ferner auf die Bereitstellung eines Inhalationspulvers, welches die Applikation des inhalierfähigen Wirkstoffanteils bei möglichst geringer Variabilität erlaubt.

Wenn auch nicht ausschließlich, so aber doch insbesondere bei der Applikation von Inhalationspulvern mittels pulver-haltiger Kapseln, spielt das Entleerungsverhalten aus dem Pulverreservoir (das Behältnis, aus dem heraus das Wirkstoff-haltige Inhalationspulver zur inhalativen Applikation freigesetzt wird) eine bedeutsame Rolle. Wird die Pulverformulierung aus dem Pulverreservoir aufgrund eines nur geringen bzw. schlechten Entleerungsverhaltens nur in geringem Maße freigesetzt, bleiben bedeutsame Mengen des wirkstoffhaltigen Inhalationspulvers im Pulverreservoir (z.B. der Kapsel) zurück und können durch den Patienten nicht therapeutisch nutzbar gemacht werden. Dies hat zur Konsequenz, daß die Dosierung des Wirkstoffs in der Pulvermischung erhöht werden muß, damit die ausgebrachte Wirkstoffmenge zur Erzielung des therapeutisch gewünschten Effekts ausreichend hoch ist.
Vor diesem Hintergrund ist es eine weitere Aufgabe der vorliegenden Erfindung, eine Inhalationspulver bereitzustellen, welches ferner durch ein sehr gutes Entleerungsverhalten gekennzeichnet ist.

### Detaillierte Beschreibung der Erfindung

Überraschenderweise wurde gefunden, daß die eingangs genannten Aufgaben durch die nachstehend beschriebenen, erfindungsgemäßen pulverförmigen Zubereitungen für die Inhalation (Inhalationspulver) gelöst werden.

Dementsprechend zielt die vorliegende Erfindung auf Inhalationspulver, enthaltend als alleinigen Wirkstoff ein Hydrat der Verbindung der Formel 1 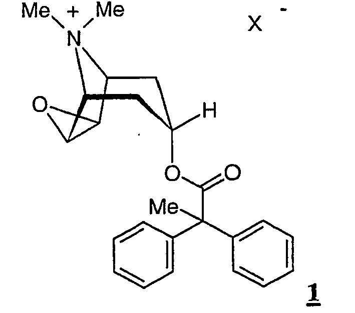 worin X⁻ für ein pharmazeutisch verträgliches Anion steht, im Gemisch mit einem physiologisch unbedenklichen Hilfsstoff, dadurch gekennzeichnet, daß der Hilfsstoff eine mittlere Teilchengröße von 12 bis 35 µm dass der Hilfstoff einen 10%- Feinanteil von 0,5 bis 6 µm aufweist.

Dabei wird unter der mittleren Teilchengröße im hier verwendeten Sinne der 50 % - Wert aus der Volumenverteilung gemessen mit einem Laserdiffraktometer nach der Trockendispersionsmethode verstanden.

Bevorzugt sind Inhaltionspulver, die als alleinigen wirkstoff eine Verbindung der Formel **1** enthalten, in der das Anion X⁻ ausgewählt ist aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat.

Bevorzugt gelangen die Salze der Formel **1** zur Anwendung, worin X - ein Anion ausgewählt aus der Gruppe Chlorid, Bromid, 4-Toluolsulfonat und Methansulfonat bedeutet.

Erfindungsgemäß besonders bevorzugt im Rahmen der vorliegenden Erfindung sind diejenigen Formulierungen, die die Verbindung der Formel **1**, in der X - für Bromid steht, als alleinigen Wirkstoff enthalten.

Bezugnahmen auf die Verbindung der Formel 1 schließen im Rahmen der vorliegenden Erfindung stets alle möglichen amorphen und kristallinen Modifikationen dieser Verbindung mit ein.

Eine im Rahmen der vorliegenden Erfindung erfolgende Bezugnahme auf die Verbindung **1'** ist als Bezugnahme auf das in den Salzen **1** enthaltene pharmakologisch aktive Kation der nachstehenden Formel anzusehen

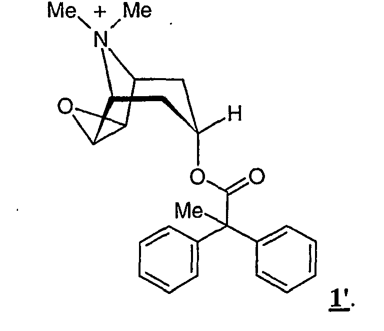

Der Wirkstoffgehalt in den erfindungsgemäßen Inhalationspulvern liegt in einem Bereich von 0,0008 bis 33%, bezogen auf das pharmakologisch aktive Kation **1'**. Im Rahmen der vorliegenden Erfindung sind Gehaltsangaben in Prozent stets als Gewichtsprozent zu verstehen, soweit nichts Gegenteiliges spezifisch hervorgehoben wird.

Erfindungsgemäß bevorzugt sind Inhalationspulver, die 0,008 bis 20,6% **1'** enthalten. Besonders bevorzugte Inhalationspulver enthalten **1'** in einer Menge von etwa 0,025 bis 9,9%, bevorzugt 0,05 bis 6,6%, besonders bevorzugt 0,07 bis 3,3%. Erfindungsgemäß von besonderer Bedeutung sind schließlich Inhalationspulver, die etwa 0,08 bis 2,5% an Kation **1'** enthalten.

Aus dem Gehalt an pharmakologisch aktivem Kation **1'** läßt sich der Gehalt der Verbindungen der Formel **1** (= Kation **1'** plus Anion X') je nach Wahl des entsprechenden Anions X⁻ zwanglos berechnen. Steht X⁻ beispielsweise für das erfindungsgemäß besonders bevorzugte Anion Bromid, können die erfindungsgemäßen Inhalationspulver zwischen 0,001 und 40 %, bevorzugt 0,01 bis 25 % der Verbindung **1** (in form des Bromids) enthalten. Erfindungsgemäß von besonderem Interesse sind Inhalationspulver, die etwa 0,03 bis 12%, bevorzugt 0,06 bis 8%, besonders bevorzugt 0,08 bis 4% der Verbindung **1** in Form des Bromids enthalten. Erfindungsgemäß von besonderer Bedeutung sind Inhalationspulver, die etwa 0,1 bis 3% der Verbindung der Formel **1** (in Form des Bromids) enthalten.

Die erfindungsgemäßen Formulierungen enthalten die Verbindungen der Formel **1** als alleinigen Wirkstoff. Arzneimittelformulierungen, die neben einer Verbindung der Formel **1** weitere Wirkstoffe enthalten, sind nicht Gegenstand dieser Erfindung.

In besonders bevorzugten Inhalationspulvern ist der Hilfsstoff durch eine mittlere Teilchengröße von 13 bis 30 µm gekennzeichnet.

Ausführungsformen der Erfindung sind ferner dadurch gekennzeichnet, dass der Hilfsstoff einen 10%-Feinanteil von 0,5 bis 6 µm aufweist.
Dabei wird unter dem 10 % - Feinanteil im hier verwendeten Sinne der 10 % - Wert aus der mit einem Laserdiffraktometer nach der Trockendispersionsmethode gemessenen Volumenverteilung verstanden.

Insbesondere solche Inhalationspulver sind erfindungsgemäß besonders bevorzugt, in denen der 10%-Feinanteil etwa 1 bis 4 µm, bevorzugt etwa 1,5 bis 3 µm beträgt.

Besonders bevorzugt sind darüberhinaus solche Inhaltionspulver, in denen der Hilfsstoff eine spezifischen Oberfläche von 0,1 bis 2 m²/g aufweist. Unter spezifischer Oberfläche wird im Sinne der Erfindung die massenspezifische Pulveroberfläche verstanden, berechnet aus der N₂-Absorptions-Isotherme, die bei dem Kochpunkt von flüssigem Stickstoff beobachtet wird (Methode von Brunauer, Emmett und Teller). Erfindungsgemäß bevorzugt sind ferner solche Inhalationspulver, in denen der Hilfsstoff eine spezifische Oberfläche zwischen 0,2 und 1,5 m²/g, bevorzugt zwischen 0,3 und 1,0 m²/g besitzt.

Die Hilfsstoffe, die im Sinne der vorliegenden Erfindung zur Anwendung gelangen, werden bevorzugt durch geeignetes Mahlen und/oder Sieben nach gängigen, im Stand der Technik bekannten Verfahren hergestellt. Gegebenenfalls handelt es sich bei den erfindungsgemäß zum Einsatz gelangenden Hilfsstoffen auch um Hilfsstoffgemische, die durch Mischen von Hilfsstofffraktionen unterschiedlicher mittlerer Teilchengröße erhalten werden.

Als physiologisch unbedenkliche Hilfsstoffe, die zur Darstellung der im Rahmen der erfindungsgemäßen Inhaletten zur Anwendung gelangenden Inhalationspulver Verwendung finden können, seien beispielsweise genannt Monosaccharide (z.B. Glucose, Fructose, Arabinose), Disaccharide (z.B. Lactose, Saccharose, Maltose, Trehalose), Oligo- und Polysaccharide (z.B. Dextrane, Dextrine, Maltodextrin, Stärke, Cellulose), Polyalkohole (z.B. Sorbit, Mannit, Xylit), Cyclodextrine (z. B. α-Cyclodextrin, β-Cyclodextrin, χ-Cyclodextrin, Methyl-β-Cyclodextrin, Hydroxypropyl-β-Cyclodextrin), Aminosäuren (z.B. Argininhydrochlorid) oder auch Salze (z.B. Natriumchlorid, Calciumcarbonat). Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt als Hilfsstoff Lactose, höchst bevorzugt Lactosemonohydrat zur Anwendung.

Für die erfindungsgemäßen Pulverformulierungen werden bevorzugt Hilfsstoffe hoher Kristallinität verwendet. Diese Kristallinität kann anhand der beim Lösen des Hilfsstoffs freiwerdenden Enthalpie (Lösungsenthalpie) beurteilt. Im Falle des erfindungsgemäß besonders bevorzugt zum Einsatz gelangenden Hilfsstoffs Lactosemonohydrat, wird vorzugsweise Lactose verwendet, die durch eine Lösungsenthalpie von ≥ 45 J/g, bevorzugt von ≥ 50 J/g, besonders bevorzugt von ≥ 52 J/g gekennzeichnet ist.

Die erfindungsgemäßen Inhalationspulver sind entsprechend der der vorliegenden Erfindung zugrunde liegenden Aufgabe gekennzeichnet durch ein hohes Maß an Homogenität im Sinne der Einzeldosierungsgenauigkeit. Diese liegt in einem Bereich von < 8% , bevorzugt < 6% , besonders bevorzugt < 4%.

Nach Einwaage der Ausgangsmaterialien erfolgt die Herstellung der Inhalationspulver aus dem Hilfsstoff und dem Wirkstoff unter Verwendung von im Stand der Technik bekannten Verfahren an. Hierbei sei beispielsweise auf die Offenbarung der WO 02/30390 verwiesen. Die erfindungsgemäßen Inhalationspulver sind dementsprechend beispielsweise gemäß der nachfolgend beschriebenen Vorgehensweise erhältlich. Bei den nachstehend beschriebenen Herstellverfahren werden die genannten Komponenten in den Gewichtsanteilen eingesetzt, wie sie in den zuvor beschriebenen Zusammensetzungen der Inhalationspulver beschrieben wurden.

Zunächst werden der Hilfsstoff und der Wirkstoff in einen geeigneten Mischbehälter eingebracht. Der verwendete Wirkstoff weist eine mittlere Teilchengröße von 0,5 bis 10 µm, vorzugsweise von 1 bis 6 µm, besonders bevorzugt von 2 bis 5 µm auf. Die Zugabe des Wirkstoffs und des Hilfsstoffs erfolgt vorzugsweise über ein Sieb oder einen Siebgranulator mit einer Maschenweite von 0,1 bis 2 mm, besonders bevorzugt 0,3 bis 1 mm, höchst bevorzugt 0,3 bis 0,6 mm. Vorzugsweise wird der Hilfsstoff vorgelegt und anschließend der Wirkstoff in den Mischbehälter eingebracht. Bevorzugt erfolgt bei diesem Mischverfahren die Zugabe der beiden Komponenten portionsweise. Besonders bevorzugt ist das abwechselnde, schichtweise Einsieben der beiden Komponenten. Der Mischvorgang des Hilfsstoffs mit dem Wirkstoff kann bereits während der Zugabe der beiden Komponenten erfolgen. Vorzugsweise wird allerdings erst nach dem schichtweisen Einsieben der beiden Bestandteile gemischt.

Sofern der in das oben beschriebene Verfahren eingesetzte Wirkstoff nicht bereits nach seiner chemischen Herstellung in einer kristallinen Form erhältlich ist, die vorstehend genannte Teilchengrößen aufweist, kann er durch Mahlen in die Teilchengrößen überführt werden, die den vorstehend genannten Parametern genügen (sogenanntes Mikronisieren).

Zur Durchführung des Mikronisierprozesses können gängige Mühlen zum Einsatz gelangen. Bevorzugt wird die Mikronisierung dabei unter Feuchtigkeitsausschluß durchgeführt, besonders bevorzugt unter Einsatz eines entsprechenden Inertgases, wie beispielsweise Stickstoff. Als besonders bevorzugt hat sich die Verwendung von Luftstrahlmühlen erwiesen, in denen die Zerkleinerung des Mahlguts durch Aufeinanderprallen der Partikel miteinender sowie Aufprall der Partikel auf die Wände des Mahlbehälters erfolgt. Als Mahlgas gelangt erfindungsgemäß bevorzugt Stickstoff zur Anwendung. Das Mahlgut wird mittels des Mahlgases unter spezifischen Drücken (Mahldruck) gefördert. Im Rahmen der vorliegenden Erfindung wird der Mahldruck üblicherweise auf einen Wert zwischen etwa 2 und etwa 8 bar, bevorzugt zwischen etwa 3 und etwa 7 bar, besonders bevorzugt zwischen etwa 3,5 und etwa 6,5 bar eingestellt. Der Eintrag des Mahlgutes in die Luftstrahlmühle erfolgt mittels des Speisegases unter spezifischen Drücken (Speisedruck). Im Rahmen der vorliegenden Erfindung hat sich eine Speisedruck zwischen etwa 2 und etwa 8 bar, bevorzugt zwischen etwa 3 und etwa 7 bar, besonders bevorzugt zwischen etwa 3,5 und etwa 6 bar bewährt. Als Speisegas gelangt vorzugsweise ebenfalls ein Inertgas, besonders bevorzugt ebenfalls Stickstoff zur Anwendung. Die Zufuhr des Mahlguts (kristalline Verbindung der Formel **1**) kann dabei in einer Förderrate von etwa 5-35 g/min, vorzugsweise mit etwa 10-30 g/min erfolgen. Beispielsweise und ohne den Gegenstand der Erfindung darauf zu beschränken, hat sich als eine mögliche Ausführungsform einer Luftstrahlmühle das folgende Gerät bewährt: 2-Zoll Microniser mit Mahlring 0,8 mm-Bohrung, Firma Sturtevant Inc., 348 Circuit Street, Hanover, MA 02239, USA. Unter Verwendung dieses Geräts wird der Mahlprozess vorzugsweise mit folgenden Mahlparametern durchgeführt:
Mahldruck: etwa 4,5 - 6,5 bar; Speisedruck: etwa 4,5 - 6,5 bar: Zufuhr des Mahlguts: etwa 17 - 21 g/min.

Gegebenenfalls kann es vorteilhaft sein, den erfindungsgemäßen Inhalationspulvern eine feinere Hilfsstofffraktion, die eine mittlere Teilchengröße von 1 - 9 µm aufweist, beizumischen. Solche Inhalationspulver, die neben dem Wirkstoff der Formel **1** ein Hilfsstoffgemisch enthalten, welches neben dem eingangs genannten Hilfsstoff der mittleren Teilchengröße von 10 - 50µm ferner eine spezifisch zugesetzte Hilfsstofffraktion einer mittleren Teilchengröße von 1-9 µm aufweist, werden vorzugsweise dadurch hergestellt, dass zunächst die beiden Hilfsstofffraktionen gemischt werden und anschließend diesem Hilfsstoffgemisch der Wirkstoff beigemischt wird. Geeignete Verfahren zur Herstellung solcher Wirkstoffformulierungen sind im Stand der Technik bekannt (z.B. WO 02/30390, WO 03/017970, WO 03/017979). Werden Hilfsstoffgemische aus gröberen und feineren Hilfststoffanteilen eingesetzt, beträgt der Anteil von feinerem Hilfsstoff an der Gesamthilfsstoffmenge vorzugsweise 1 bis 20%. Werden Hilfststoffgemische aus gröberen und feineren Hilfststoffanteilen eingesetzt, weist die gröbere Hilfsstofffraktion hinsichtlich mittlerer Teilchengröße, spezifischer Oberfläche oder auch Kristallinität bevorzugt die vorstehend genannten Eigenschaften auf, während die beigemischte feinere Hilfsstofffraktion bevorzugt durch eine mittlere Teilchengröße von von 2 bis 8µm, besonders bevorzugt von 3 bis 7µm gekennzeichnet ist. Darüberhinaus beträgt der Anteil der feinen Hilfsstofffraktion an der Gesamthilfsstoffmenge bei solchen gegebenenfalls zum Einsatz gelangenden Inhalationspulvern bevorzugt 3 bis 15%, besonders bevorzugt 5 bis 10%.

Die vorliegende Erfindung betrifft ferner die Verwendung der erfindungsgemäßen Inhalationspulver zur Herstellung eines Arzneimittels zur Behandlung von Atemwegserkrankungen, insbesondere zur Behandlung von COPD und/oder Asthma.

Die erfindungsgemäßen Inhalationspulver können beispielsweise mittels Inhalatoren appliziert werden, die eine einzelne Dosis aus einem Vorrat mittels einer Meßkammer (z.B. gemäß US 4570630A) oder über andere apparative Vorrichtungen (z.B. gemäß DE 36 25 685 A) dosieren.

Alternativ dazu und erfindungsgemäß von gleichrangiger Bedeutung, können die erfindungsgemäßen Inhalationspulver auch mittels Inhalatoren appliziert werden, die das Inhalationspulver in mehreren, einzeln verpackten Dosen enthalten (Pre-Metered Dry Powder Inhaler).

Alternativ dazu und erfindungsgemäß von gleichrangiger Bedeutung, können die erfindungsgemäßen Inhalationspulver auch in Kapseln abgefüllt werden, die in Inhalatoren wie beispielsweise in der WO 94/28958 beschrieben, zur Anwendung gelangen.

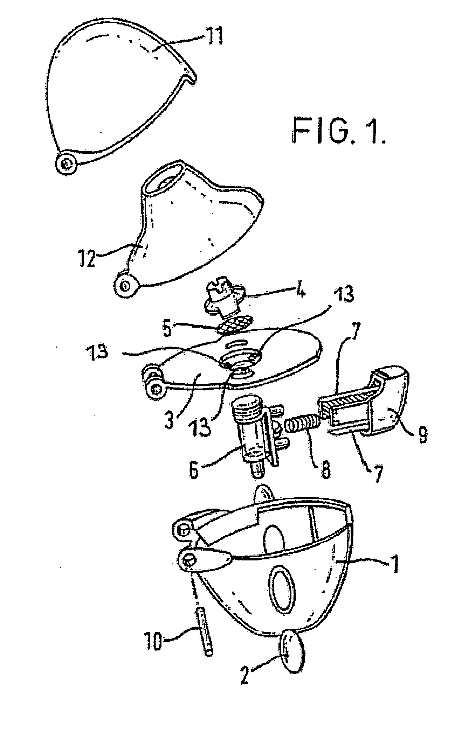

Besonders bevorzugt werden die das erfindungsgemäße Inhalationspulver enthaltenden Kapseln mit einem Inhalator appliziert, wie er in Figur 1 dargestellt ist. Dieser Inhalator ist gekennzeichnet durch ein Gehäuse 1, enthaltend zwei Fenster 2, ein Deck 3, in dem sich Lufteinlaßöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, ein über eine Achse 10 klappbar mit dem Gehäuse 1, dem Deck 3 und einer Kappe 11 verbundenes Mundstück 12, sowie Luftdurchlaßlöcher 13 zur Einstellung des Strömungswiderstandes.

Die vorliegende Erfindung betrifft ferner die Verwendung der erfindungsgemäßen Inhalationspulver zur Herstellung eines Arzneimittels zur Behandlung von Atemwegserkrankungen, insbesondere zur Behandlung von COPD und/oder Asthma, dadurch gekennzeichnet, daß der vorstehend beschriebene, in Figur 1 dargestellte Inhalator zur Anwendung gelangt.

Für die Anwendung der erfindungsgemäßen Inhalationspulver mittels pulverhaltiger Kapseln können Kapseln unterschiedlichsten Materials verwendet werden. Unter Kapselmaterial wird im Rahmen der vorliegenden Erfindung dabei das Material verstanden, aus dem die Hülle der Inhalationskapsel gefertigt ist. Das Kapselmaterial ist erfindungsgemäß bevorzugt ausgewählt aus der Gruppe bestehend aus Gelatine, Cellulosederivaten, Stärke, Stärkederivaten, Chitosan und synthetischen Kunststoffen.

Wird Gelatine als Kapselmaterial verwendet, so kann diese im Gemisch mit anderen Zusätzen ausgewählt aus der Gruppe bestehend aus Polyethylenglycol (PEG), bevorzugt PEG 3350, Glycerol, Sorbitol, Propylenglycol, PEO-PPO-Blockcopolymeren und anderen Polyalkoholen und Polyethern zum Einsatz kommen. Besonders bevorzugt wird im Rahmen der vorliegenden Erfindung Gelatine im Gemisch mit PEG, bevorzugt PEG 3350, verwendet. Besonders bevorzugt enthält eine erfindungsgemäße Gelatine-Kapsel PEG in einem Anteil von 1-10% (Gew-%), bevorzugt 3-8 %. Besonders bevorzugte GelatineKapseln enthalten PEG in einem Anteil von 4-6%, wobei ein PEG-Anteil von etwa 5% erfindungsgemäß höchstbevorzugt ist.

Werden Cellulosederivate als Kapselmaterial verwendet, so ist die Verwendung von Hydroxypropylmethylcellulose, Hydroxypropylcellulose, Methylcellulose, Hydroxymethylcellulose und Hydroxyethylcellulose bevorzugt. Besonders bevorzugt wird in diesem Fall Hydroxypropylmethylcellulose (HPMC), besonders bevorzugt HPMC 2910 als Kapselmaterial eingesetzt.

Werden als Kapselmaterial synthetische Kunststoffe eingesetzt, so sind diese erfindungsgemäß bevorzugt ausgewählt aus der Gruppe bestehend aus Polyethylen, Polycarbonat, Polyester, Polypropylen und Polyethylenterephthalat. Besonders bevorzugt sind als synthetische Kunststoffmaterialien Polyethylen, Polycarbonat oder Polyethylenterephthalat. Wird Polyethylen als eines der erfindungsgemäß besonders bevorzugten Kapselmaterialien verwendet, gelangt vorzugsweise Polyethylen mit einer Dichte zwischen 900 und 1000 kg/m³, bevorzugt von 940 - 980 kg/m³, besonders bevorzugt von etwa 960 - 970 kg/m³ (high-density Polyethylen) zur Anwendung. Die synthetischen Kunststoffe im Sinne der Erfindung können vielseitig mittels dem im Stand der Technik bekannten Herstellverfahren verarbeitet werden. Bevorzugt im Sinne der Erfindung wird die spritzgusstechnische Verarbeitung der Kunststoffe. Besonders bevorzugt wird die Spritzgusstechnik unter Verzicht auf die Verwendung von Formtrennmitteln. Dieses Herstellverfahren ist wohldefiniert und durch eine besonders gute Reproduzierbarkeit gekennzeichnet.

Ein weiterer Aspekt der vorliegenden Erfindung betrifft vorstehend genannte Kapseln, die vorstehend genanntes erfindungsgemäßes Inhalationspulver enthalten. Diese Kapseln können etwa 1 bis 20mg, bevorzugt etwa 3 bis 15, besonders bevorzugt etwa 4 bis 6mg Inhalationspulver enthalten.

Ferner betrifft die vorliegende Erfindung ein Inhalationskit, bestehend aus einer oder mehrerer der vorstehend beschriebenen, durch einen Gehalt an erfindungsgemäßem Inhalationspulver gekennzeichneten Kapseln in Verbindung mit dem Inhalator gemäß Figur 1.

Die vorliegende Erfindung betrifft ferner die Verwendung der vorstehend genannten, durch einen Gehalt an erfindungsgemäßem Inhalationspulver gekennzeichneten Kapseln, zur Herstellung eines Arzneimittels zur Behandlung von Atemwegserkrankungen, insbesondere zur Behandlung von COPD und/oder Asthma.

Die Darstellung von befüllten Kapseln, die die erfindungsgemäßen Inhalationspulver enthalten, erfolgt nach im Stand der Technik bekannten Verfahren durch Befüllung der leeren Kapseln mit den erfindungsgemäßen Inhalationspulvern.

Die folgenden Beispiele dienen einer weitergehenden Erläuterung der vorliegenden Erfindung.

### Ausgangsmaterialien

### I) Hilfsstoff:

In den nachfolgenden Beispielen wird als Hilfsstoff Lactose-Monohydrat verwendet. Dieser kann beispielsweise von der Firma Borculo Domo Ingredients, Borculo/NL unter der Produktbezeichnung *Lactochem Extra Fine Powder* bezogen werden. Die erfindungsgemäßen Spezifikationen für die Teilchengröße und die spezifische Oberfläche werden von dieser Lactosequalität erfüllt. Ferner weist diese Lactose die vorstehend genannten, für Lactose erfindungsgemäß bevorzugten Lösungsenthalpie-Werte auf.

### II) Mikronisierung der Verbindung der Formel 1, in der X⁻ Bromid bedeutet:

Das gemäß der WO 02/32899 erhältliche kristalline 2,2-Diphenylpropionsäurescopinester-Methobromid (Verbindung der Formel **1** mit X' = Bromid) wird mit einer Luftstrahlmühle vom Typ 2-Zoll Microniser mit Mahlring 0,8 mm-Bohrung, Firma Sturtevant Inc., 348 Circuit Street, Hanover, MA 02239, USA mikronisiert. Unter Verwendung von Stickstoff als Mahlgas werden dabei beispielsweise die folgenden Mahlparameter eingestellt:
Mahldruck: 5,5 bar; Speisedruck: 5,5 bar;
Zufuhr: 19 g/min.

### Meßmethoden:

### I) Partikelgrößenbestimmung des Mikronisats der Verbindung der Formel 1 (X⁻ =Bromid):

### Messgerät und Einstellungen:

Die Bedienung der Geräte erfolgte in Übereinstimmung mit den Bedienungsanleitungen des Herstellers.

| | |
|---|---|
| Messgerät: | Laser-Beugungs-Spektrometer (HELOS), Sympatec |
| Dispergiereinheit: | Trockendispergierer RODOS mit Saugtrichter,Sympatec |
| Probenmenge: | 200 mg ± 150 mg |
| Produktzufuhr: | Schwingrinne Vibri, Fa. Sympatec |
| Frequenz d. Vibrationsrinne: | bis 100 % ansteigend |
| Dauer der Probenzufuhr: | 15 bis 25 sek. (im Fall von 200 mg) |
| Brennweite: | 100 mm (Messbereich: 0,9 - 175 µm) |
| Messzeit/Wartezeit: | ca. 15 s (im Fall von 200 mg) |
| Zykluszeit: | 20 ms |
| Start/Stop bei: | 1 % auf Kanal 28 |
| Dispergiergas: | Druckluft |
| Druck: | 3 bar |
| Unterdruck: | maximal |
| Auswertemodus: | HRLD |

### Probenvorbereitung / Produktzufuhr:

Ca. 200 mg der Prüfsubstanz werden auf einem Kartenblatt eingewogen.
Mit einem weiteren Kartenblatt werden alle größeren Agglomerate zerstoßen.
Das Pulver wird dann auf der vorderen Hälfte der Schwingrinne (ab ca. 1 cm vom vorderen Rand) fein verteilt aufgestreut.
Nach dem Start der Messung wird die Frequenz der Schwingrinne so variiert, dass die Zufuhr der Probe möglichst kontinuierlich erfolgt. Die Produktmenge darf aber auch nicht zu groß sein, damit eine ausreichende Dispergierung erreicht wird.

### II) Partikelgrößenbestimmung der Laktose:

### Messgerät und Einstellungen

Die Bedienung der Geräte erfolgte in Übereinstimmung mit den Bedienungsanleitungen des Herstellers.

| | |
|---|---|
| Messgerät: | Laser-Beugungs-Spektrometer (HELOS), Sympatec |
| Dispergiereinheit: | Trockendispergierer RODOS mit Saugtrichter, Sympatec |
| Probenmenge: | 200 mg ± 100 mg |
| Produktzufuhr: | Vibrationsrinne Typ VIBRI , Sympatec |
| Frequenz d. Vibrationsrinne: | 100 % ansteigend |
| Brennweite: | 200 mm (Messbereich: 1,8 - 350 µm) |
| Messzeit/Wartezeit: | ca. 10 s (im Falle von 200 mg) |
| Zykluszeit: | 10 ms |
| Start/Stop bei: | 1 % auf Kanal 28 |

| Dispergiergas: | Druckluft |
|---|---|
| Druck: | 3 bar |
| Unterdruck: | maximal |
| Auswertemodus: | HRLD |

### Probenvorbereitung / Produktzufuhr:

Ca. 200 mg der Prüfsubstanz werden auf einem Kartenblatt eingewogen. Mit einem weiteren Kartenblatt werden alle größeren Agglomerate zerstoßen. Das Pulver wird in die Vibrationsrinne überführt. Es wird ein Abstand von 1.2 bis 1.4 mm zwischen Vibrationsrinne und Trichter eingestellt. Nach dem Start der Messung wird die Amplitudeneinstellung der Schwingrinne möglichst kontinuierlich auf 100 % gegen Ende der Messung gesteigert.

### III) Bestimmung der spezifischen Oberfläche der Laktose (Mehrpunkt-BET-Methode):

### Prinzip

Die Bestimmung der spezifischen Oberfläche erfolgt, indem die Pulverprobe einer Stickstoffatmosphäre bei unterschiedlichen Drücken ausgesetzt wird. Durch Abkühlung der Probe erfolgt eine Kondensation der Stickstoffmoleküle auf der Oberfläche der Partikel. Die kondensierte Stickstoffmenge wird über den Druckabfall im System bestimmt und die spezifische Oberfläche der Probe über den Flächenbedarf von Stickstoff und der Probeneinwaage berechnet.

Die Bedienung der Geräte erfolgte in Übereinstimmung mit den Bedienungsanleitungen des Herstellers.

### Meßgerät und Einstellungen

| | |
|---|---|
| Meßgerät | Tri Star Multi Point BET, Fa. Micromeritics |
| Ausheizstation: | VacPrep 061, Fa. Micromeritics |
| Ausheizen: | ca. 12h / 40°C |
| Sample tube: | ½ inch; use filler rod |
| Analysis Condition: | 10 point BET surface 0,1 to 0,20 p/p0 |
| Absolute P. tolerance: | 5,0 mmHg |
| rel. P. tolerance: | 5,0 % |
| Evacuation rate: | 50,0 mmHg/sec. |
| Unvestticted evac f.: | 10,0 mmHg |
| Evac. time: | 0,1 hours |
| Free Space: | Lower Dewar, time: 0,5 h |
| Equilibration interv.: | 20 sec |
| Min. equl. delay: | 600 sec |
| Adsorptive: | Nitrogen |

### IV) Bestimmung der Lösungswärme der Laktose (Lösungsenthalpie) E_{c}:

Die Bestimmung der Lösungsenthalpie erfolgt mittels eines Lösungskalorimeter 2225 *Precision Solution Calorimeter* der Fa. Thermometric :
Die Lösungswärme wird anhand der - infolge des Löseprozesses - auftretende Temperaturänderung und der aus der Basislinie berechneten systembedingten Temperaturänderung berechnet. Vor und nach dem Ampullenbruch wird jeweils eine elektrische Kalibrierung mit einem integrierten Heizwiderstand genau bekannter Leistung durchgeführt. Hierbei wird eine bekannte Wärmeleistung über einen festgelegten Zeitraum an das System abgegeben und der Temperatursprung ermittelt.

### Messgerät und Einstellungen

| | |
|---|---|
| Lösungskalorimeter: | 2225 Precision Solution Calorimeter, |
| | Fa. Thermometric |
| Reaktionszelle: | 100 ml |
| Thermistorwiderstand: | 30,0 kΩ (bei 25 °C) |
| Rührergeschwindigkeit: | 500 U/min |
| Thermostat: | Thermostat des 2277 Thermal Activity Monitor TAM, Fa. Thermometric |
| Temperatur: | 25 °C ± 0.0001 °C (über 24h) |
| Meßampullen: | Crushing ampoules 1 ml, Fa. Thermometric |
| Dichtung: | Silikonstopfen und Bienenwachs, Fa. Thermometric |
| Einwaage: | 40 bis 50 mg |
| Lösemittel: | Wasser, chemisch rein |
| Volumen Lösemittel: | 100 ml |
| Badtemperatur: | 25°C |
| Temperaturauflösung: | High |
| Starttemperatur: | -40mK (± 10mK) temperature-offset |
| Interface: | 2280-002 TAM accessory interface 50 Hz, Fa. Thermometric |
| Software: | SolCal V 1.1 für WINDOWS |
| Auswertung: | Automatische Auswertung mit Menüpunkt CALCULATION/ ANALYSE EXPERIMENT. (Dynamik der Basislinie ; Kalibrierung nach dem Ampullenbruch). |

### Elektrische Kalibrierung:

Die elektrische Kalibrierung erfolgt während der Messung, einmal vor und einmal nach dem Ampullenbruch. Zur Auswertung wird die Kalibrierung nach dem Ampullenbruch herangezogen.

| | |
|---|---|
| Wärmemenge: | 2,5 J |
| Heizleistung: | 500 mW |
| Heizdauer: | 10 s |
| Dauer der Basislinien: | 5 min (vor und nach Heizen) |

### Darstellung der erfindungsgemäßen Pulverformulierungen:

### I) Apparatives

Zur Herstellung der Inhalationspulver können beispielsweise die folgenden Maschinen und Geräte Verwendung finden:
Mischbehälter bzw. Pulvermischer: Turbulamischer 2 L, Typ 2C; Hersteller Willy A. Bachofen AG, CH-4500 Basel
Handsieb: 0,135 mm Maschenweite

Die Befüllung der leeren Inhaltionskapseln mittels wirkstoffhaltigem Inhaltionspulver kann händisch oder maschinell erfolgen. Es können nachstehende Geräte verwendet werden.

### Kapselfüllmaschine:

MG2, Typ G100, Hersteller: MG2 S.r.1,I-40065 Pian di Macina di Pianoro (BO), Italien

### Beispiel 1:

### Pulvermischung :

Zur Herstellung der Pulvermischung werden 297,0 g Hilfsstoff und 3,0 g mikronisierte Verbindung der Formel **1** (in der X⁻ Bromid bedeutet) eingesetzt. In den daraus erhaltenen 300 g Inhalationspulver beträgt der Wirkstoffanteil 1 % (bezogen auf Verbindung der Formel **1** mit X⁻ Bromid). Dieser Wirkstoffanteil entspricht bei Bezugnahme auf das pharmakologisch aktive Kation **1'** einem Anteil bzw. Gehalt von etwa 0,825 %.

Über ein Handsieb mit einer Maschenweite von 0,315 mm werden in einen geeigneten Mischbehälter ca. 40-45 g Hilfsstoff vorgelegt. Anschließend werden abwechselnd mikronisierte Verbindung der Formel **1** (mit X⁻ = Bromid) in Portionen von ca. 450-550 mg und Hilfsstoff in Portionen von etwa 40-45 g schichtweise eingesiebt. Die Zugabe des Hilfsstoffs und des Wirkstoffs erfolgt in 7 bzw. 6 Schichten.

Die eingesiebten Bestandteile werden anschließend gemischt (Mischen: 900 Umdrehungen). Die Endmischung wird noch zweimal über ein Handsieb gegeben und anschließend jeweils gemischt (Mischen: 900 Umdrehungen).

Gemäß oder in Analogie zu der in Beispiel 1 beschriebenen Vorgehensweise können folgende Inhalationspulver erhalten werden:

### Beispiel 2:

| | |
|---|---|
| Wirkstoff **1** (mit X⁻ = Bromid) | 3,000g |
| Lactose Monohydrat*⁾: | 297,000g |
| Total: | 300,000g |
| mittlere Teilchengröße: | 17,9 µm; |
| 10%-Feinanteil: | 2,3 µm; |
| spezifische Oberfläche: | 0,61 m²/g; |

| | |
|---|---|
| *) der Hilfsstoff ist durch die folgenden Parameter gekennzeichnet: | |

### Beispiel 3:

| | |
|---|---|
| Wirkstoff **1** (mit X⁻ = Bromid) | 3,000g |
| Lactose Monohydrat*): | 297,000g |
| Total: | 300,000g |
| mittlere Teilchengröße: | 18,5 µm; |
| 10%-Feinanteil: | 2,2 µm; |
| spezifische Oberfläche: | 0,83 m²/g; |

| | |
|---|---|
| *) der Hilfsstoff ist durch die folgenden Parameter gekennzeichnet: | |

### Beispiel 4:

| | |
|---|---|
| Wirkstoff **1** (mit X⁻ = Bromid) | 3,000g |
| Lactose Monohydrat^{*)}: | 297,000g |
| Total: | 300,000g |
| mittlere Teilchengröße: | 21,6 µm; |
| 10%-Feinanteil: | 2,5 µm; |
| spezifische Oberfläche: | 0,59 m²/g; |

| | |
|---|---|
| *⁾ der Hilfsstoff ist durch die folgenden Parameter gekennzeichnet: | |

### Beispiel 5:

| | |
|---|---|
| Wirkstoff **1** (mit X⁻ = Bromid) | 3,000g |
| Lactose Monohydrat*⁾: | 297,000g |
| Total: | 300,000g |
| mittlere Teilchengröße: | 16,0 µm; |
| 10%-Feinanteil: | 2,0 µm; |
| spezifische Oberfläche: | 0,79 m²/g; |

| | |
|---|---|
| *⁾ der Hilfsstoff ist durch die folgenden Parameter gekennzeichnet: | |

### Beispiel 6:

| | |
|---|---|
| Wirkstoff **1** (mit X⁻ = Bromid) | 6,000g |
| Lactose Monohydrat*⁾: | 294,000g |
| Total: | 300,000g |
| mittlere Teilchengröße: | 17,9 µm; |
| 10%-Feinanteil: | 2,3 µm; |
| spezifische Oberfläche: | 0,61 m²/g; |

| | |
|---|---|
| *⁾ der Hilfsstoff ist durch die folgenden Parameter gekennzeichnet: | |

### Beispiel 7:

| | |
|---|---|
| Wirkstoff **1** (mit X⁻ = Bromid) | 6,000g |
| Lactose Monohydrat*⁾: | 294,000g |
| Total: | 300,000g |
| mittlere Teilchengröße: | 18,5 µm; |
| 10%-Feinanteil: | 2,2 µm; |
| spezifische Oberfläche: | 0,83 m²/g; |

| | |
|---|---|
| *⁾ der Hilfsstoff ist durch die folgenden Parameter gekennzeichnet: | |

### Beispiel 8:

| | |
|---|---|
| Wirkstoff **1** (mit X⁻ = Bromid) | 1,500g |
| Lactose Monohydrat*⁾: | 298,500g |
| Total: | 300,000g |
| mittlere Teilchengröße: | 17,9 µm; |
| 10%-Feinanteil: | 2,3 µm; |
| spezifische Oberfläche: | 0,61 m²/g; |

| | |
|---|---|
| *⁾ der Hilfsstoff ist durch die folgenden Parameter gekennzeichnet: | |

### Beispiel 9:

| | |
|---|---|
| Wirkstoff **1** (mit X⁻ = Bromid) | 1,500g |
| Lactose Monohydrat*⁾: | 298,500*g |
| Total: | 300,000g |
| mittlere Teilchengröße: | 18,5 µm; |
| 10%-Feinanteil: | 2,2 µm; |
| spezifische Oberfläche: | 0,83 m²/g; |

| | |
|---|---|
| *⁾ der Hilfsstoff ist durch die folgenden Parameter gekennzeichnet: | |

### Beispiel 10:

| | |
|---|---|
| Wirkstoff **1** (mit X⁻ = Bromid) | 6,000g |
| Dextrose Monohydrat*⁾: | 294,000g |
| Total: | 300,000g |
| mittlere Teilchengröße: | 18,5 µm; |
| 10%-Feinanteil: | 2,2 µm; |

| | |
|---|---|
| *⁾ der Hilfsstoff ist durch die folgenden Parameter gekennzeichnet: | |

### Beispiel 11:

| | |
|---|---|
| Wirkstoff **1** (mit X⁻ = Bromid) | 1,500g |
| Dextrose Monohydrat^{*)}: | 298,500g |
| Total: | 300,000g |
| mittlere Teilchengröße: | 17,9 µm; |
| 10%-Feinanteil: | 2,3 µm; |

| | |
|---|---|
| *⁾ der Hilfsstoff ist durch die folgenden Parameter gekennzeichnet: | |

### Beispiel 12:

| | |
|---|---|
| Wirkstoff **1** (mit X⁻ = Bromid) | 3,000g |
| Dextrose Monohydrat*⁾: | 297,000g |
| Total: | 300,000g |
| mittlere Teilchengröße: | 18,5 µm; |
| 10%-Feinanteil: | 2,2 µm; |

| | |
|---|---|
| *⁾ der Hilfsstoff ist durch die folgenden Parameter gekennzeichnet: | |

Die oben genannten Bespielformulierungen können in geeigneten Mengen in geeignete Packmittel, z. B. Polyethylenkapseln abgefüllt werden oder sind direkt in Mehrdosispulverinhalatoren einsetzbar.

## Patentansprüche

1. Inhalationspulver, enthaltend als alleinigen Wirkstoff ein Hydrat der Verbindung der Formel **1** 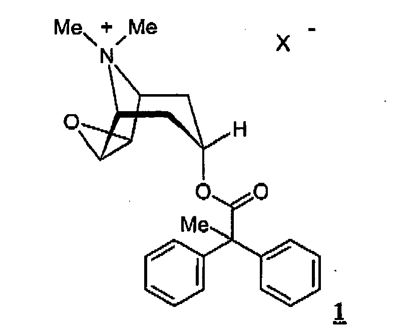 worin X⁻ für ein pharmazeutisch verträgliches Anion steht, im Gemisch mit einem physiologisch unbedenklichen Hilfsstoff, **dadurch gekennzeichnet,, dass** der Hilfsstoff eine mittlere Teilchengröße von 12 bis 35 µm und dass der Hilfsstoff einen 10%-Feinanteil von 0,5 bis 6 µm aufweist.

2. Inhalationspulver nach Anspruch 1, **dadurch gekennzeichnet, daß** das Anion X⁻ ausgewählt ist aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat.

3. Inhalationspulver nach Anspruch 1 oder 2, **dadurch gekennzeichnet, dass** der Wirkstoffgehalt bezogen auf das pharmakologisch aktive Kation der Formel **1'** 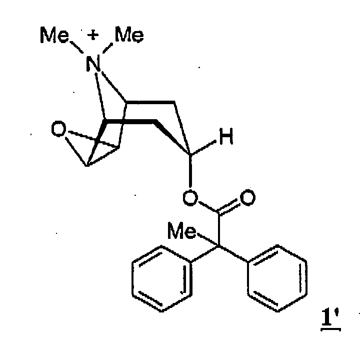 zwischen 0,0008 und 33 % beiträgt.

4. Inhalationspulver nach Anspruch 1, 2 oder 3, **dadurch gekennzeichnet, daß** der physiologisch unbedenkliche Hilfsstoff ausgewählt ist aus der Gruppe der Monosaccharide, der Disaccharide, der Oligo- und Polysaccharide, der Polyalkohole, der Cyclodextrine, der Aminosäuren oder auch der Salze.

5. Inhalationspulver nach einem der Ansprüche 1 bis 4, **dadurch gekennzeichnet, dass** der Hilfsstoff eine spezifischen Oberfläche von 0,1 bis 2 m²/g aufweist.

6. Verwendung eines Inhalationspulvers gemäß einem der Ansprüche 1 bis 5 zur Herstellung eines Arzneimittels zur Behandlung von Atemwegserkrankungen, insbesondere zur Behandlung von COPD und/oder Asthma.

7. Kapsel enthaltend ein Inhalationspulver nach einem der Ansprüche 1 bis 5.

8. Kapsel nach Anspruch 7, **dadurch gekennzeichnet, daß** das Kapselmaterial aus synthetischem Kunststoff besteht.

9. Inhalationskit bestehend aus einer Kapsel nach einem der Ansprüche 7 oder 8 und einem Inhalator, der zur Applikation von Inhaltionspulvern aus pulverhaltigen Kapseln verwendet werden kann.

10. Inhalationskit nach Anspruch 9, **dadurch gekennzeichnet, daß** der Inhalator **gekennzeichnet ist durch** ein Gehäuse 1, enthaltend zwei Fenster 2, ein Deck 3, in dem sich Lufteinlaßöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, ein über eine Achse 10 klappbar mit dem Gehäuse 1, dem Deck 3 und einer Kappe 11 verbundenes Mundstück 12, sowie Luftdurchlaßlöcher 13 zur Einstellung des Strömungswiderstandes.

## Claims

1. Inhalable powder, containing as sole active substance a hydrate of the compound of formula 1 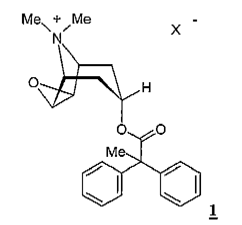 wherein X⁻ denotes a pharmaceutically acceptable anion, in admixture with a physiologically acceptable excipient, **characterised in that** the excipient has an average particle size of 12 to 35 µm and the excipient has a 10% fine content of 0.5 to 6 µm.

2. Inhalable powder according to claim 1, **characterised in that** the anion X⁻ is selected from the group consisting of chloride, bromide, iodide, sulphate, phosphate, methanesulphonate, nitrate, maleate, acetate, citrate, fumarate, tartrate, oxalate, succinate, benzoate and p-toluenesulphonate.

3. Inhalable powder according to claim 1 or 2, **characterised in that** the content of active substance based on the pharmacologically active cation of formula 1' 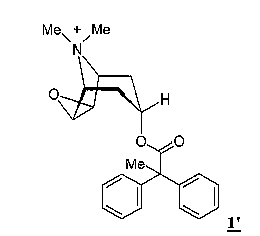 is between 0.0008 and 33 %.

4. Inhalable powder according to claim 1, 2 or 3, **characterised in that** the physiologically acceptable excipient is selected from among the monosaccharides, disaccharides, oligo- and polysaccharides, the polyalcohols, cyclodextrins, amino acids or the salts thereof.

5. Inhalable powder according to one of claims 1 to 4, **characterised in that** the excipient has a specific surface area of 0.1 to 2 m²/g.

6. Use of an inhalable powder according to one of claims 1 to 5 for preparing a pharmaceutical composition for the treatment of respiratory complaints, particularly for the treatment of COPD and/or asthma.

7. Capsule containing an inhalable powder according to one of claims 1 to 5.

8. Capsule according to claim 7, **characterised in that** the capsule material consists of synthetic plastics.

9. Inhalation kit consisting of a capsule according to one of claims 7 or 8 and an inhaler which may be used for administering inhalable powders from powderfilled capsules.

10. Inhalation kit according to claim 9, **characterised in that** the inhaler is **characterised by** a housing 1 containing two windows 2, a deck 3 in which there are air inlet ports and which is provided with a screen 5 secured via a screen housing 4, an inhalation chamber 6 connected to the deck 3 on which there is a push button 9 provided with two sharpened pins 7 and movable counter to a spring 8, a mouthpiece 12 which is connected to the housing 1, the deck 3 and a cover 11 via a spindle 10 to enable it to be flipped open or shut, as well as air through-holes 13 for adjusting the flow resistance.

## Revendications

1. Poudre pour l'inhalation contenant comme seul principe actif un hydrate du composé de formule **1** 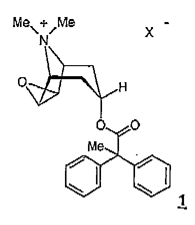 où X⁻ représente un anion pharmaceutiquement acceptable, en mélange avec un adjuvant physiologiquement acceptable, **caractérisée en ce que** l'adjuvant présente une taille de particule moyenne de 12 à 35 µm et **en ce que** l'adjuvant présente une fraction fine à 10 % de 0,5 à 6 µm.

2. Poudre pour l'inhalation selon la revendication 1 **caractérisée en ce que** l'anion X⁻ est choisi dans le groupe consistant en chlorure, bromure, iodure, sulfate, phosphate, méthanesulfonate, nitrate, maléate, acétate, citrate, fumarate, tartrate, oxalate, succinate, benzoate et p-toluènesulfonate.

3. Poudre pour l'inhalation selon la revendication 1 ou 2 **caractérisée en ce que** la teneur en principe actif rapportée au cation pharmacologiquement actif de formule **1'** 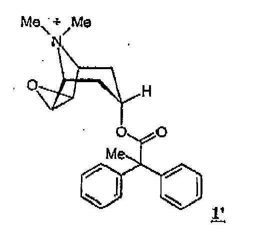 est entre 0,0008 et 33 %.

4. Poudre pour l'inhalation selon la revendication 1, 2 ou 3 **caractérisée en ce que** l'adjuvant physiologiquement acceptable est choisi dans le groupe des monosaccharides, des disaccharides, des oligo- et polysaccharides, des polyalcools, des cyclodextrines, des aminoacides ou encore des sels.

5. Poudre pour l'inhalation selon l'une des revendications 1 à 4 **caractérisée en ce que** l'adjuvant présente une surface spécifique de 0,1 à 2 m²/g.

6. Utilisation d'une poudre pour l'inhalation selon l'une des revendications 1 à 5 pour la production d'un médicament pour le traitement des maladies des voies respiratoires, en particulier pour le traitement de la COPD et/ou de l'asthme.

7. Capsule contenant une poudre pour l'inhalation selon l'une des revendications 1 à 5.

8. Capsule selon la revendication 7 **caractérisée en ce que** le matériau de la capsule consiste en matière plastique synthétique.

9. Kit pour l'inhalation consistant en une capsule selon l'une des revendications 7 et 8 et en un inhalateur qui peut être utilisé pour l'application de poudres pour l'inhalation à partir de capsules contenant une poudre.

10. Kit pour l'inhalation selon la revendication 9 **caractérisé en ce que** l'inhalateur est **caractérisé par** un boîtier 1, contenant deux fenêtres 2, un couvercle 3, dans lequel se trouvent des ouvertures d'entrée d'air et qui est muni d'un tamis 5 fixé par le biais d'un boîtier de tamis 4, une chambre d'inhalation 6 reliée au couvercle 3, sur laquelle il est prévu un bouton-poussoir 9 mobile contre un ressort 8, muni de deux aiguilles affûtées 7, un embout buccal 12 relié à pivotement par le biais d'un axe 10 au boîtier 1, au couvercle 3 et à un capuchon 11, ainsi que des trous de passage d'air 13 pour le réglage de la résistance à l'écoulement.
